# EUROPEAN PATENT APPLICATION

(11) **EP 2 482 413 A1**
(43) Date of publication of application: **01.08.2012**
(21) Application number: 12150264.5
(22) Date of filing: 05.01.2012
(51) Int. Cl.: H02H 7/18, H02J 7/00

(54) **Battery interlock system and related method**

(30) Priority: 07.01.2011 US 986859
(71) Applicant: Tyco Health Group LP, Mansfield, MA 02048 (US)
(72) Inventor: Krapohl, James E., Broomfield, CO Colorado 80020 (US)
(74) Representative: HOFFMANN EITLE

(57) **Abstract**

An interlock system and related method includes a battery assembly (102), and first and second modules (202, 204). The battery assembly (102) has first and second power contacts (114, 112), and a sense contact (116). The battery assembly includes one or more cells (106), a standby circuit (108), and an interface circuit (110). The standby circuit (108) electrically couples to the first power contact (114) and the sense contact (116) for generating a standby signal in the absence of a detected external electrical coupling between the first power contact (114) and the sense contact (116). The interface circuit (110) electrically couples to the second power contact (112) and to the at least one cell (106). The interface circuit (110) is adapted to electrically decouple the second power contact (112) from the at least one cell (106) as a function of the standby signal. The first module (202) is attachable to the battery assembly (102) and has a first pair of contacts (208, 214) adapted to couple with the first power contact (114) and the sense contact (116) of the battery assembly. The first module also has a second pair of contacts (220, 222) electrically coupled to the first pair of contacts (208, 210). The second module (204) is attachable to the first module (202), The second module (204) has a third pair of contacts (226, 228) adapted to couple with the second pair of contacts (220, 222). The contacts of the third pair of contacts (226, 228) are electrically coupled together (230).

## Description

### BACKGROUND

### Technical Field

The present disclosure relates to surgical instruments. More particularly, the present disclosure relates to surgical instruments having a battery interlock.

### Background of Related Art

Typically, a portable surgical instrument includes a battery assembly that couples or "latches" to the portable surgical instrument. In an ideal scenario, the battery assembly remains coupled or "latched" to the portable surgical instrument during the entirety of the surgical procedure. In certain instances, the battery assembly has to be uncoupled or "unlatched" from the portable surgical instrument. For example, the battery assembly may have to be unlatched from the surgical instrument for sterilization (or re-sterilization), charging (or recharging), etc. During the time the battery assembly is uncoupled with the portable surgical instrument, contacts of the battery assembly may be exposed. Exposed and live contacts are susceptible to shorting caused by conductive objects.

### SUMMARY

In an embodiment of the present disclosure, an interlock system includes a battery assembly, and first and second modules. The battery assembly has first and second power contacts, a sense contact, one or more cells, a standby circuit, and an interface circuit. The first power contact may be in electrical communication with a negative terminal of the at least one cell; and the second power contact may be in electrical communication with a positive terminal of the at least one cell. The standby circuit is electrically coupled to the first power contact and the sense contact. The standby circuit generates a standby signal in the absence of a detested external electrical coupling between the first power contact and the sense contact. For example, the first and second modules provide the external electrical coupling between the first power contact and sense contact when the first and second modules, and the battery assembly are electro-mechanically coupled. The interface circuit electrically couples to the second power contact and to the one or more cells. The interface circuit electrically decouples the second power contact from the one or more cells as a function of the standby signal.

The first module is attachable to the battery assembly and has a first pair of contacts adapted to couple with the first power contact and the sense contact of the battery assembly. The first module also has a second pair of contacts electrically coupled to the first pair of contacts. The second module is attachable to the first module, and the second module has a third pair of contacts adapted to couple with the second pair of contacts. All of the contacts of the third pair of contacts are electrically coupled together.

The first module further includes a power receiver contact, a power supply contact, and an electrical conductor. The power receiver contact electrically couples to the first power contact of the battery assembly. The power contact supplies power. The electrical conductor supplies power between the power receiver contact and the power supply contact. The second module includes another power supply contact that electrically couples to the power supply contact of the first module to receive power therefrom.

In another embodiment of the present disclosure, the first module may be a surgical assembly (e.g., a disposable instrument) and the second module may be a generator attachable to the surgical assembly. The generator supplies mechanical vibration to an end effector. Additionally or alternatively, the generator is an electrosurgical generator adapted to supply electrosurgical energy to one or more electrodes disposed on the end effector.

In yet another embodiment of the present disclosure, the battery assembly include a PNP transistor. The PNP transistor has base, emitter, and collector terminals. The base terminal is operatively coupled to the sense contact of the battery assembly. The emitter terminal is operatively coupled to a positive terminal of the one or more cells. And, the collector terminal is operatively coupled to the second power contact of the battery assembly. The PNP transistor couples and decouples a power contact from the battery cells.

In another embodiment of the present disclosure, a variable-impedance device electrically couples between the second power contact and the one or more cells to provide a variable impedance therebetween. The variable-impedance device sets the impedance to a high impedance state in response to the standby signal. The variable-impedance device may be a, contractor, a relay, an insulated gate bipolar transistor, or a silicon-controlled rectifier.

In yet another embodiment of the present disclosure, a surgical system includes a disposable instrument, a generator, and a battery assembly. The disposable instrument has first and second communication contacts and may include an end effector. The generator has a third communication contact. The generator is adapted to attach to the disposable instrument such that the third communication contact of the generator couples to the first communication contact of the disposable instrument when the generator attaches to the disposable instrument. The generator may be an electrosurgical generator and/or an ultrasonic generator.

The battery assembly attaches to the disposable instrument and includes one or more power contacts, one or more cells, a fourth communication contact, a standby circuit, and an interface circuit. The fourth communication contact couples to the second communication contact of the disposable instrument when the battery attaches to the disposable instrument. The standby circuit electrically couples to the fourth communication contact and communicates with the generator and the disposable instrument to determine when the disposable instrument, the generator, and the battery assembly are electro-mechanically coupled. The interface circuit electrically couples to one or more power contacts and to one or more cells, The interface circuit electrically decouples one or more power contacts from one or more cells as a function of the determination by the standby circuit of when the disposable instrument, the generator, and the battery assembly are electro-mechanically coupled. One or more of the power contacts may be coupled to a negative terminal of the cells and/or a positive terminal of the cells.

In yet another embodiment of the present disclosure, a method for assembling a surgical system includes: assembling together a battery assembly, a disposable instrument, and a generator; determining if the battery assembly, the disposable instrument, and the generator are eicctro-mechanically coupled; and activating a power contact of the battery assembly in response to the determination that the battery assembly, the disposable instrument, and the generator are electro-mechanically coupled. The method may also include: communicating between the battery assembly and the disposable instrument to determine if the battery assembly, the disposable instrument, and the generator are electro-mechanically coupled; communicating between the battery assembly and the generator to determine if the battery assembly, the disposable instrument, and the generator are electro-mechanically coupled; and reducing an impedance between at least one cell of the battery assembly and the power contact in response to the determination that the battery assembly, the disposable instrument, and the generator are electro-mechanically coupled.

In yet another embodiment of the present disclosure, a battery interlock system includes a plurality of modules and a battery assembly. The plurality of modules each has one or more communication contacts. The battery assembly includes one or more power contacts and a communication contact. The battery assembly operatively communicates with each of the plurality of modules via each respective one or more communication contacts to determine if all of the plurality of modules are electro-inechanically coupled with the battery assembly. The battery assembly activates the one or more power contacts when all of the plurality of modules is electro-mechanically coupled with the battery assembly.

### BRIEF DESCRIPTION OF THE DRAWINGS

Various embodiments of the present disclosure are described herein with reference to the drawings wherein:
Figs. 1A and 1B are schematic views of an interlock system including a battery assembly and a module according to an embodiment of the present disclosure;
Figs. 2A and 2B are schematic views of an interlock system including a battery assembly and two modules according to an embodiment of the present disclosure;
Fig. 3 is a schematic view a battery assembly according to another embodiment of the present disclosure;
Fig. 4 is a schematic view of an interlock system including a battery assembly and two modules according to another embodiment of the present disclosure;
Fig. 5 is a side, perspective view of a battery powered surgical instrument configured for use with a removable battery assembly according to yet another embodiment of the present disclosure; and
Fig. 6 shows a method for assembling a surgical system according to one embodiment of the present disclosure.

### DETAILED DESCRIPTION OF THE EMBODIMENTS

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one ordinary skill in the relevant art to variously employ the present disclosure in virtually any appropriately detailed structure.

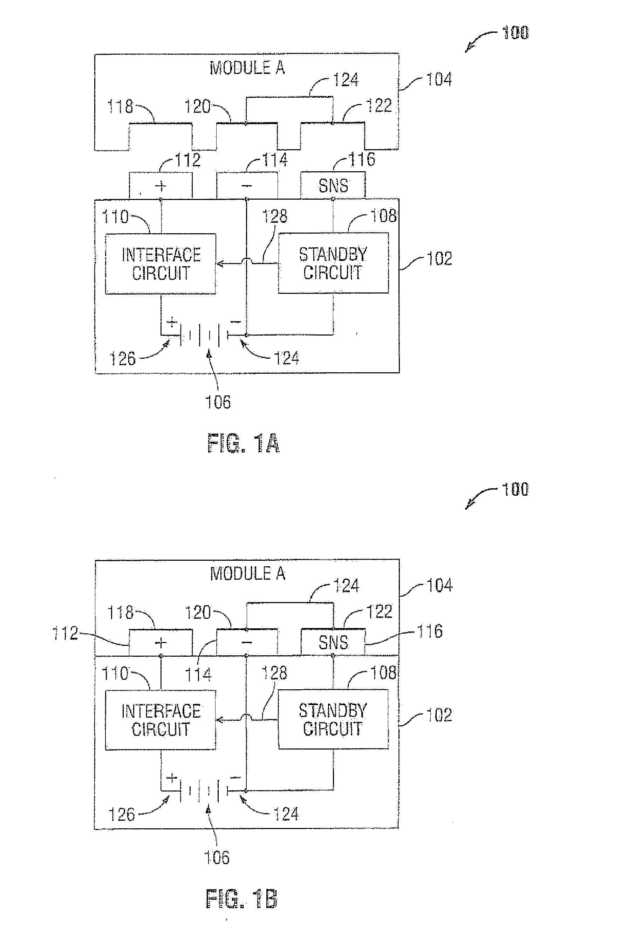

Figs. 1A and 1B are schematic views of an interlock system 100 including a battery assembly 102 and a module 104 according to an embodiment of the present disclosure. Fig. 1A shows the battery assembly 102 unattached to the nodule 104; and Fig. 1B shows the battery assembly 102 attached to the module 104 to power to the module 104 when the module is sufficiently attached thereto. The battery assembly 102 and the module 104 are keyed to prevent improper or reverse assembly.

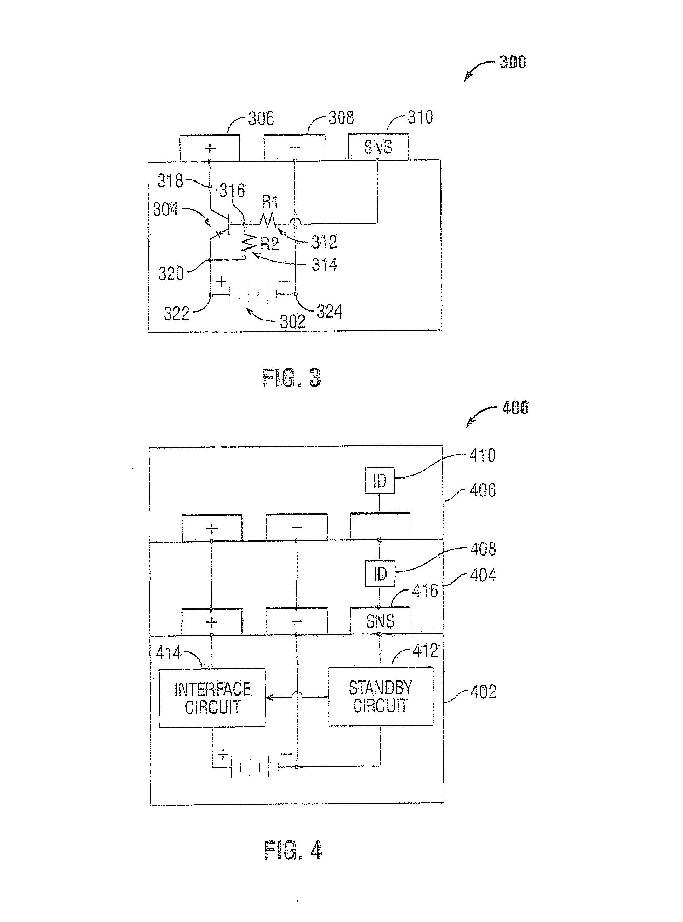

The battery assembly 102 includes battery cells 106, a standby circuit 108, and an interface circuit 110. The battery assembly 100 also includes a sense contact 116, and two power contacts, i.e., a positive contact 112 and a negative contact 114. The standby circuit 108 detects any electrical coupling between the negative contact 114 and the sense contact 116 to determine when the module 104 is attached to the battery assembly 102. The standby circuit 108 generates a standby signal when the module 104 is not attached to the battery assembly 102 as determined by the standby circuit 108. In some embodiments of the present disclosure, the standby circuit 108 and the interface circuit 110 may be integrated together as a single circuit or device (e.g., see Fig. 3).

The module 104 includes contacts 118, 120 and 122, and a conductor 124. The conductor 124 may be a wire, a conductive strip, a metallic path, a semiconductor conductive path, and/or the like. The conductor 124 provides a conductive path between contacts 120 and 122. As shown in Fig. 1B, when the module 104 couples to the battery assembly 102, the contact 120 electrically couples contact 114 to contact 122. For example, the standby circuit 108 can determine that the negative contact 114 and the sense contact 116 reside at the same electric potential as the negative terminal 124 of the battery cells 106. Additionally or alternatively, the battery assembly 102 can determine if the positive contact 112 and the sense contact 116 reside at the same electric potential as the positive terminal 126 of the battery cells 106.

The standby circuit 108 detects when the negative contact 114 electrically couples to the sense contact 122. As shown in Fig. 1B, the contact 120 electrically couples to the ground contact 114 when the module 104 couples to the battery assembly 102; and the sense contact 116 electrically couples to the contact 122 when the module 104 couples to the battery assembly 102. The conductor 124 in the module 104 completes the connection such that the ground contact 114 is in electrical communication with the sense contact 116 when the module 104 attaches to the battery assembly 102. The standby circuit 108 can sense the external coupling between the ground contact 114 and the sense contact 116 to determine that the module 104 and the battery assembly 102 are electro-mechanically coupled.

The standby circuit 108 provides a standby signal to the interface circuit 110 via a data path 128. The standby signal can be positive logic or negative logic. For example, a standby signal can generate a high logic voltage, e.g., 5 Volts, to indicate to the interface circuit 110 that the module 104 is not attached, or the standby circuit 108 can generate a low logic level, e.g., 0 Volts, to indicate to the interface circuit 110 that the module 104 is not attached. In some embodiments of the present disclosure, a standby signal is the absence of an enable or "on" signal from the standby circuit 108.

The interface circuit 110 electrically couples to the positive terminal 126 of the battery cells 106, and to the positive contact 112. Also, the interface circuit 110 receives the standby signal from the data path 128. The interface circuit 110 decouples and couples the positive terminal 126 from the positive contact 112 as a function of the standby signal from the standby circuit 108. The interface circuit 110 may decouple the positive contact 112 from the positive terminal 126 by varying the impedance therebetween.

For example, in some embodiments of the present disclosure, the interface circuit 110 includes a variable impendance device coupled between the positive terminal 126 and the positive contact 112 to vary the impedance therebetween. When the interface circuit 110 receives a standby signal from the standby circuit 108, the interface circuit 110 signals to the variable impedance device to enter into a high impendance state, e.g., infinity or sufficiently high impedance. The variable impedance device may be a contactor, a relay, an insulated gate bipolar transistor, a silicon-controlled rectifier, and the like.

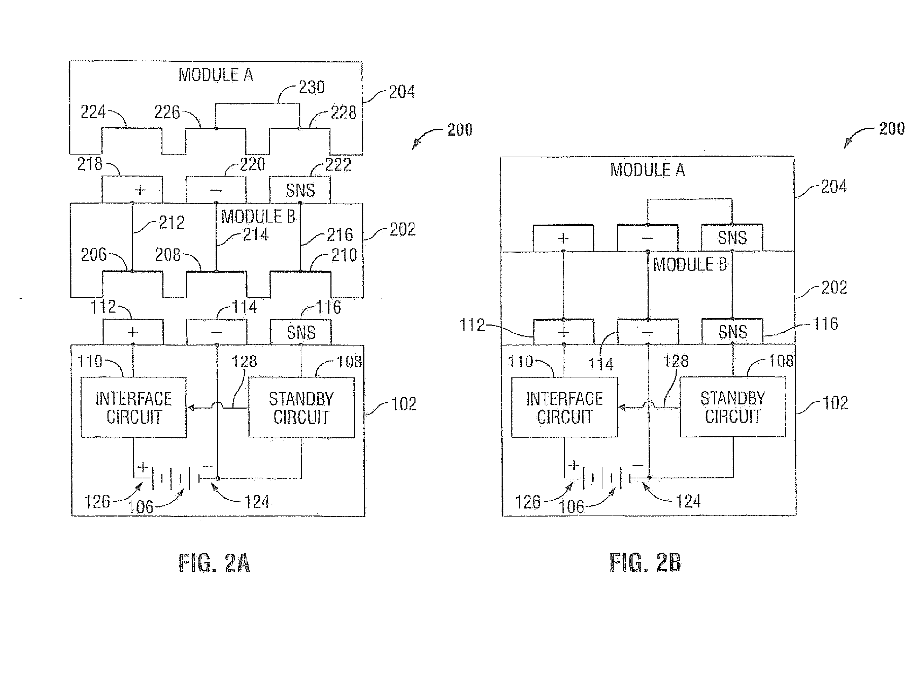

Referring to the drawings, Figs. 2A and 2B are schematic views of an interlock system 200 including a battery assembly 102 and two modules, i.e., modules 202 and 204, according to an embodiment of the present disclosure. The battery assembly 102 of the battery system 200 may be similar or identical to the battery assembly 102 of Figs. 1A and 1B. The interlock system 200 includes the battery assembly 102, the module 202, and the module 204. The battery assembly 102, the module 202 and/or the module 204 may be keyed to prevent improper or reverse assembly. The interface circuit 110 electrically couples the positive terminal 126 of the battery cells 106 to the positive contact 112 when the standby circuit 108 determines that the battery assembly 102, the module 202, and the module 204 are electro-mechanically coupled.

Fig. 2A shows the battery assembly 102, the module 202, and the module 204 electro-mechanically uncoupled. If the module 202 is connected to the battery assembly 102, and the module 204 is not connected to the module 202, then the interlock system 200 is uncoupled. Likewise, if the module 204 is attached to the module 202, but the module 202 is not attached to the battery assembly 102, then the interlock system 200 is also electro-mechanically uncoupled.

Fig. 2B shows the interlock system 200 coupled together. The interlock system 200 is coupled together when the module 204 is coupled to the module 202, and the module 202 is coupled to the battery assembly 102. In some embodiments, the modules 202 and 204 attach to the battery assembly 102 to be fully coupled together (not depicted in Figs. 2A and 2B).

The standby circuit 108 senses electrical coupling between contacts 114 and 116 to determine when the interlock system 200 is electro-mechanically coupled. Module 202 includes contacts, 206, 208, 210, 218, 220, and 222. Module 202 includes conductors 212, 214, and 216. Conductor 212 couples to contact 206 and to contact 218; conductor 214 couples to contact 208 and to contact 220; and conductor 216 couples to contact 210 to contact 222.

When the module 202 is attached to the battery assembly 102, and the module 204 is attached to the module 202, the standby circuit 108 determines that the interlock system 200 is electro-mechanically coupled. When the interface circuit 110 does not receive a standby signal from the standby circuit 108, the interface circuit 110 enables the coupling between the positive terminal 126 of the battery cells 106 to the positive contact 112. When the interlock system 200 is electro-mechanically coupled, there is a conductive path from the negative contact 114 through the conductor 214 to the contact 220, through the contact 226, through the conductive path 230, through the contact 228, through the contact 222, through the conductive path 216, through the contact 210, through the sense contact 116, and finally to the standby circuit 108. The standby circuit 108 therefore detects when the interlock system 200 is clectto-mechanically coupled when there is a complete conductive path between the negative contact 114 and the sense contact 116

Fig. 3 is a schematic view of a battery assembly 300 according to an embodiment of the present disclosure. The battery assembly 300 includes battery cells 302, a transistor 304, and contacts 306, 308 and 310. The battery assembly 300 may be keyed to prevent improper or reverse coupling to a module (e.g., modules 104, 202, or 204 of Figs. 1A-2B). The battery assembly 300 also includes resistor R1 312, and resistor R2 314. Fig. 3 shows the battery assembly 300 having an interface circuit and standby circuit integrated together. Specifically, the battery assembly 300 includes the transistor 304, the resistor R1 312 and the resistor R2 314 that together from an integrated standby circuit 108 and interface circuit 110 of Figs. 1A-1B.

The transistor 304 includes a base terminal 316, a collector terminal 318 and an emitter terminal 320. When there is no coupling between contacts 308 and 310, there is high impedance between the positive terminal 322 of the battery cells 302 and the positive contact 306. When the negative contact 308 is in electrical communication with the sense contact 310, the base terminal 316 of the transistor electrically couples to the negative terminal 324 of the battery cells 302. The transistor 304 turns "on" when the base terminal 316 electrically couples to the negative terminal 324 through the resistor R1 312, e.g., when there is a conductive path between the negative contact 308 and the sense contact 310. When the transistor 304 is on, the impedance between the positive terminal 322 and the contact 306 is much lower thereby allowing the battery 302 to supply sufficient electrical energy through the terminal 306 to power a module, e.g., modules 202 and 204 of Figs. 2A-2B.

Fig. 4 is a schematic view of another embodiment of an interlock system 400 including a battery assembly 402 and two modules, i.e., module 404 and module 406, according to an embodiment of the present disclosure. The battery assembly 402, the module 404, and/or the module 406 may be keyed to prevent improper or reverse assembly. Module 404 includes an ID 408 and module 406 includes an ID 410. Fig 4 shows the module 404, the module 406, and the battery assembly 402 when electro-mechanically coupled. When the interlock system 400 is electro-mechanically coupled, the standby circuit 412 can communicate with the ID 408 and the ID 410 to determine when the interlock system 400 is electro-mechanically coupled. The standby circuit 412 provides a standby signal to the interface circuit, 414 when the interlock system 400 is not electro-mechanically coupled.

The ID 408 and the ID 410 maybe a ROM, an EEPROM, or other memory that contains a numerical value representing a module, a type of module, a serial number, or security code value. The standby circuit 412 can communicate with the ID 408 and the ID 410 using a serial communication protocol, e.g., firewire, USB, IC2, and the like. Additionally or alternatively, IDs 408 and 410 can respond using an encrypted response using an encryption key stored therein.

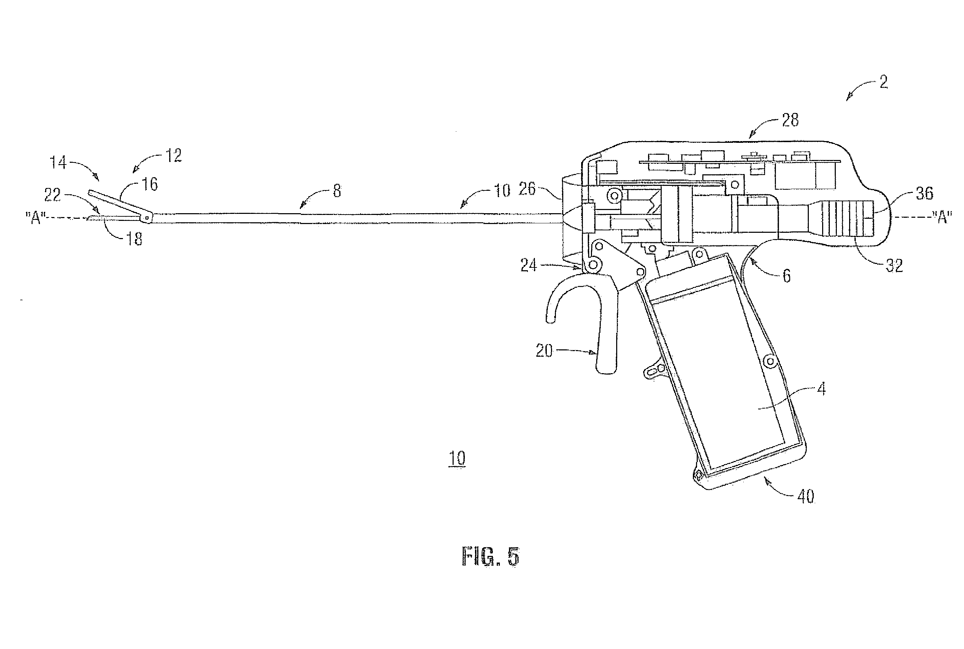

Fig. 5 shows a battery powered surgical instrument 2 configured for use with a removable battery assembly 4 according to an embodiment of the present disclosure. Battery assembly 4 may be configured for use with a variety of battery-powered instruments including, but not limited to, electrosurgical forceps, electrosurgical staplers, ultrasonic surgical devices, etc. For illustrative purposes, the battery assembly 4 is described in terms of use with a portable ultrasonic surgical instrument 2. The battery assembly 4 may be the battery assembly 102 of Figs. 1A, 1B, 2A, or 2B, the battery assembly 300 of Fig. 3, or the battery assembly 402 of Fig. 4.

Instrument 2 includes a disposable instrument or housing 6. Housing 6 is configured to house one or more components, e.g., a waveguide, electrical circuitry that is configured for electrical communication with the battery assembly 4, etc., of the instrument 2. The housing 6 may be a module of the battery interlock systems disclosed herein, e.g., module 104 of Figs. 1A-1B, modules 202 or 204 of Figs. 2A-2B, modules 404 or 406 of Fig. 4. A proximal end of housing 6 is configured to releasably couple to a generator 28 and the battery assembly 4, described in greater detail below. The generator 28 may also be a module of a battery interlock systems disclosed herein, e.g., module 104 of Figs. 1A-1B, modules 202 or 204 of Figs. 2A-2B, modules 404 or 406 of Fig. 4. The generator 28 selectively engages with the disposable instrument or housing 6. A distal end of the housing 6 is configured to support and/or couple to a shaft 8.

The shaft 8 extends from the housing 6 and defines a longitudinal axis "A-A" therethrough. A shaft rotation knob 26 is operably coupled to the shaft 8 and is configured to rotate the shaft 8. A proximal end 10 of the shaft 8 is operably coupled to the housing 6 and a distal end 12 of the shaft 8 is operably coupled to a surgical probe or end effector 14.

The end effector 14 includes a pair of jaw members 16 and 18. Jaw member 16 pivots about the jaw 18 (and/or the distal end 12 of the shaft 8) and moves relative thereto when a lever or movable handle 20 moves proximally. More particularly, jaw member 16 is movable from an open position for positioning tissue between the jaw members 16 and 18, to a clamping position for grasping tissue between the jaw members 16 and 18 and against jaw member 18, Jaw member 18 serves as an active or oscillating blade and is configured to affect tissue. To this end, jaw member 18 includes an ultrasonic member (not shown) that is operably coupled to a transducer 32, and an operating surface 22 configured to effect tissue. In the illustrated embodiment, the operating surface 22 is configured to transect, dissect, seal, and/or coagulate tissue upon actuation of an activation button 24.

Activation button 24 places the instrument 2 in two modes of operation, a low-power mode of operation and a high-power mode of operation. More particularly, activation button 24 is depressable to a first position for delivering low power to the active jaw 18 and a second position for delivering high-power to the active jaw 18. In the first position, one or more audio or visual indicators may indicate to the user that the activation button 24 is in the low-power mode. For example, and in one particular embodiment, an audio indicator may include a low-pitch, slow pulsating tone that indicates to a user that the activation button 24 is in the first position. Likewise, one or more audio or visual indicators may indicate to a user that the activation button is in the high-power mode, e.g., an audio indicator may include a high-pitch, fast pulsating tone that indicates to a user that the activation button 24 is in the second position.

A selectively removable ultrasonic generator 28 configured to convert electrical energy generated by the battery assembly 4 to ultrasonic energy to drive the active jaw member 18 operably coupled to the housing 6. As previously mentioned, the generator 28 may also including features similar to the battery interlock systems disclosed herein, e.g., features of module 104 of Figs. 1A-1B, modules 202 or 204 of Figs. 2A-2B, or modules 404 or 406 of Fig, 4. To secure the generator 28 to the housing 6, a user positions the generator 28 on a top portion of the housing 6 at a proximal end thereof.

Generator 28 includes transducer 32 that is configured to convert electrical energy to mechanical energy to produce motion at an end of a waveguide (not explicitly shown) that is in operative communication with the active jaw member 18. The generator 28 releasably couples to the disposable instrument 6. When the transducer 32 and waveguide are driven at their resonant frequency, they produce a relatively large amount of mechanical motion at the active jaw member 18. The electronics of the generator 38 converts the electrical energy from the battery 4 into a high voltage AC waveform that drives the transducer 32, In one particular embodiment, the frequency of this AC: waveform is the same as the resonant frequency of the waveguide and transducer 32. As can be appreciated, the magnitude of the AC waveform includes a value that produces the proper amount of mechanical motion.

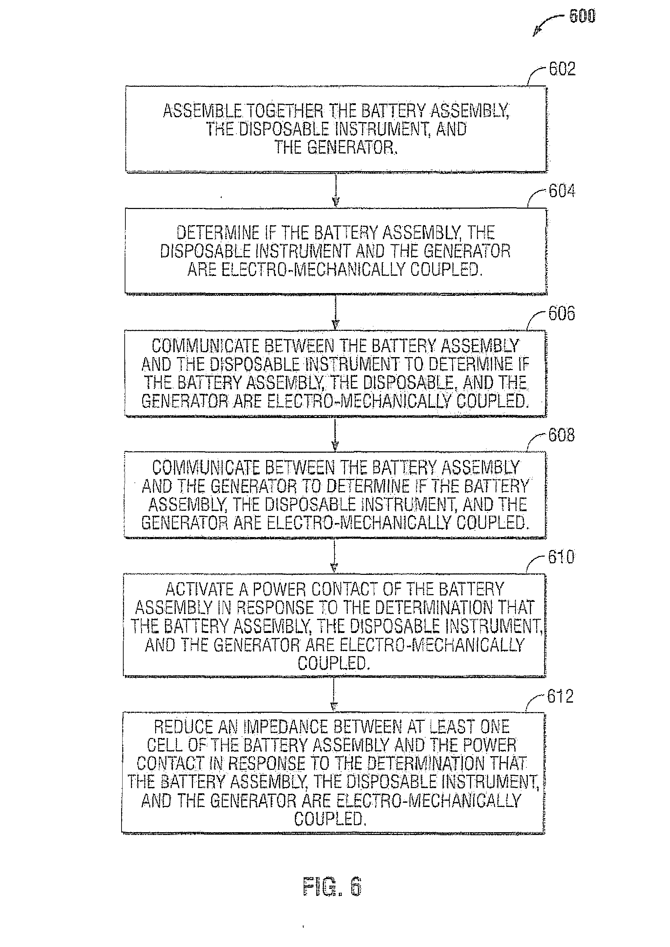

Referring to the drawings, Fig. 6 shows a method 600 for assembling a surgical system according to an embodiment of the present disclosure, Method 600 includes steps 602-612. Step 602 assembles together the battery assembly 4, the disposable instrument 6 and the generator 28 (see Fig 5). Step 604 determines if the battery assembly 4, the disposable instrument 6, and the generator 28 are electro-mechanically coupled (as shown in Fig. 5). Step 604 may make the determination using a sense contact, e.g., sense contact 116 of Figs. 1 A, 1B, 2A, or 2B, sense contact 310 of Fig. 3, or sense contact 416 of Fig. 4. Step 606 communicates between the battery assembly 4 and the disposable instrument 6 to determine if the battery assembly 4, the disposable instrument, 6, and the generator 28 are electro-mechanically coupled. Step 608 communicates between the battery assembly 4 and the generator 28 to determine if the battery assembly 4, the disposable instrument 6, and the generator 28 are electro-mechanically coupled. That is, steps 606 and 608 may be performed as sub-steps to step 604. Additionally or alternatively, steps 606 and 608 may be used by step 604 to determine if and/or when the battery assembly 4, the disposable instrument 6, and the generator 28 are electro-mechanically coupled.

Step 610 activates a power contact of the battery assembly 4 in response to the determination that the battery assembly 4, the disposable instrument 6, and the generator 28 are electro-mechanically coupled. Step 610 reduces the impedance between at least one cell, e.g., cells 106 of Figs. 1A-1B, and the power contact, e.g., the positive contact 112 of Figs. 1A-1B, in response to the battery assembly 4, the disposable instrument 6, and the generator 28 being electro-mechanically coupled. Step 612 reduces an impedance between at least one cell of the battery assembly 4 and the power contact in response to the determination that the battery assembly 4, the disposable instrument 6 and the generator 28 are electro-mechanically coupled.

The present disclosure also extends to the subject-matter defined by the following sequence of 23 numbered clauses:
1. An interlock system, comprising:
   a battery assembly having first and second power contacts, and a sense contact, the battery assembly comprising:
      at least one cell;
      a standby circuit electrically coupled to the first power contact and the sense contact, wherein the standby circuit is configured to generate a standby signal in the absence of a detected external electrical coupling between the first power contact and the sense contact; and
      an interface circuit electrically coupled to the second power contact and to the at least one cell, wherein the interface circuit is adapted to electrically
      decouple the second power contact from the at least one cell as a function
      of the standby signal;
   a first module attachable to the battery assembly, the first module having a first pair of contacts adapted to couple with the first power contact and the sense contact of the battery assembly, and a second pair of contacts electrically coupled to the first pair of contacts; and
   a second module attachable to the first module, the second module having a third pair of contacts adapted to couple with the second pair of contacts, wherein each contact of the third pair of contacts are electrically coupled together.
2. The interlock system according to clause 1, wherein the first module further comprises:
   a power receiver contact adapted to electrically couple to the first power contact of the battery assembly;
   a power supply contact adapted to supply power therethrough; and
   an electrical conductor adapted to supply power between the power receiver contact and the power supply contact.
3. The interlock system according to clause 2, wherein the second module further comprise another power supply contact adapted to electrically couple to the power supply contact of the first module to receive power therefrom.
4. The interlock system according to any preceding clause, wherein the first power contact is in electrical communication with a negative terminal of the at least one cell.
5. The interlock system according to any preceding clause, wherein the second power contact is in electrical communication with a positive terminal of the at least one cell.
6. The interlock system according to any preceding clause, wherein the first module is a surgical assembly having an end effector.
7. The interlock system according to clause 6, wherein the second module is a generator attachable to the surgical assembly.
8. The interlock system according to clause 7, wherein the generator is an ultrasonic generator adapted to supply mechanical vibration to the end effector.
9. The interlock system according to clause 7, wherein the generator is an electrosurgical generator adapted to supply electrosurgical energy to at least one electrode disposed on the end effector.
10. The interlock system according to clause 7, 8 or 9, wherein the surgical assembly is a disposable instrument.
11. The interlock system according to any preceding clause, further comprising a PNP transistor having base, emitter, and collector terminals, wherein the base terminal is operatively coupled to the sense contact of the battery assembly, the emitter terminal is operatively coupled to a positive terminal of the at least one cell, and the collector terminal is operatively coupled to the second power contact of the battery assembly.
12. The interlock system according to any preceding clause, further comprising a variable-impedance device electrically coupled between the second power contact and the at least one cell to provide an impendance therebetween, wherein the variable-impedance device is configured to set the impedance to a high impedance state in response to the standby signal.
13. The interlock system according to clause 12, wherein the variable-impedance device is one of a contactor, a relay, an insulated gate bipolar transistor, and a silicon-controlled rectifier.
14. A surgical system, comprising:
   a disposable instrument having first and second communication contacts;
   a generator having a third communication contact, and adapted to attach to the disposable instrument, wherein the third communication contact of the generator couples to the first communication contact of the disposable instrument when the generator is attached to the disposable instrument; and
   a battery assembly adapted to attach to the disposable instrument, the battery assembly comprising:
      at least one power contact;

      at least one cell;
      a fourth communication contact, wherein the fourth communication contact couples to second communication contact of the disposable instrument when the battery is attached to the disposable instrument;
      a standby circuit electrically coupled to the fourth communication contact and adapted to communicate with the generator and the disposable instrument to determine when the disposable instrument, the generator, and the battery assembly are electro-mechanically coupled; and
      an interface circuit electrically coupled to the at least one power contact and to the at least one cell, wherein the interface circuit is adapted to electrically decouple the at least one power contact from the at least one cell as a function of the determination by the standby circuit of when the disposable instrument, the generator, and the battery assembly electro-mechanically coupled.
15. The surgical system according to clause 14, wherein the at least one power contact is in electrical communication with a negative terminal of the at least one cell.
16. The surgical system according to clause 14, wherein the at least one power contact is in electrical communication with a positive terminal of the at least one cell.
17. The surgical system according to clause 14, 15 or 16, wherein the disposable instrument includes an end effector.
18. The surgical system according to any of clauses 14 to 17, wherein the generator is an electrosurgical generator.
19. The surgical system, according to any of clauses 14 to 17, wherein the generator is an ultrasonic generator.
20. A method for assembling a surgical system, the method comprising:
   assembling together a battery assembly, a disposable instrument, and a generator;
   determining if the battery assembly, the disposable instrument, and the generator are electro-mechanically coupled; and
   activating a power contact of the battery assembly in response to the determination that the battery assembly, the disposable instrument, and the generator are electro-mechanically coupled.
21. The method according to clause 20, further comprising:
   communicating between the battery assembly and the disposable instrument to determine if the battery assembly, the disposable instrument, and the generator are electro-mechanically coupled; and
   communicating between the battery assembly and the generator to determine if the battery assembly, the disposable instrument, and the generator are electro-mechanically coupled;
22. The method according to clause 20, further comprising reducing an impedance between at least one cell of the battery assembly and the power contact in response to the determination that the battery assembly, the disposable instrument, and the generator are electro-mechanically coupled.
23. A battery interlock system, comprising:
   a plurality of modules each having at least one communication contact; and
   a battery assembly including at least one power contact and a communication contact, wherein the battery assembly operatively communicates with each of the plurality of modules via the respective at least one communication contact to determine if all of the plurality of modules are electro-mechanically coupled with the battery assembly; wherein the battery assembly activates the at least one power contact when all of the plurality of modules are electro-mechanically coupled with the battery assembly.
   While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An interlock system (100), comprising:
a battery assembly (102; 300; 402) having first and second power contacts (112, 114), and a sense contact (116), the battery assembly comprising:
at least one cell (106);
a standby circuit (108) electrically coupled to the first power contact (114) and the sense contact (116), wherein the standby circuit (108) is configured to generate a standby signal in the absence of a detected external electrical coupling between the first power contact (114) and the sense contact (116); and
an interface circuit (110) electrically coupled to the second power contact (112) and to the at least one cell (106), wherein the interface circuit (110) is adapted to electrically decouple the second power contact (112) from the at least one cell (106) as a function of the standby signal;
a first module (202) attachable to the battery assembly, the first module having a first pair of contacts (208, 210) adapted to couple respectively with the first power contact (114) and the sense contact (116) of the battery assembly, and a second pair of contacts (220, 222) electrically coupled to the first pair of contacts (208, 210); and
a second module (204) attachable to the first module (202), the second module having a third pair of contacts (226, 228) adapted to couple with the second pair of contacts (220, 222), wherein the contacts (226, 228) of the third pair of contacts are electrically coupled together (230),

2. An interlock system (100), comprising:
a battery assembly (102; 300; 402) having first and second power contacts (112, 114), and a sense contact (116), the battery assembly comprising:
at least one cell (106);
a standby circuit (108) electrically coupled to the first power contact (114) and the sense contact (116), wherein the standby circuit (108) is configured to generate a standby signal in the absence of a detected external electrical coupling between the first power contact (114) and the sense contact (116); and
an interface circuit (110) electrically coupled to the second power contact (112) and to the at least one cell (106), wherein the interface circuit (110) is adapted to electrically decouple the second power contact(112) from the at least one cell (106) as a function of the standby signal;
a module (104; 204) attachable to the battery assembly, the module having a pair of contacts (120, 122; 208, 210) adapted to couple respectively with the first power contact (114) and the sense contact (116) of the battery assembly, wherein the contacts (120, 122; 226, 228) of the pair of contacts are electrically coupled together (124; 230).

3. An interlock system (100), comprising:
a battery assembly (402) having first and second power contacts (112, 114), and a sense contact (416), the battery assembly comprising:
at least one cell;
a standby circuit (412) electrically coupled to the first power contact (114) and the sense contact (416), wherein the standby circuit (412) is configured to generate a standby signal in the absence of a detected external ID signal at the sense contact (416); and
an interface circuit (414) electrically coupled to the second power contact (112) and to the at least one cell, wherein the interface circuit (414) is adapted to electrically decouple the second power contact (112) from the at least one cell (106) as a function.of the standby signal;
a first module (404) attachable to the battery assembly, the first module having a first contact adapted to couple with the sense contact (416) of the battery assembly, and a second contact electrically coupled to the first contact; and
a second module (406) attachable to the first module (404), the second module having a further contact adapted to couple with the second contact and to supply said external ID signal.

4. The interlock system according to any preceding claim, wherein the first module (202; 404) further comprises:
a power receiver contact (206) adapted to electrically couple to the second power contact (112; 306) of the battery assembly,;
a power supply contact (218) adapted to supply power therethrough; and
an electrical conductor (212) adapted to supply power between the power receiver contact (206) and the power supply contact (218).

5. The interlock system according to claim 4, wherein the second module (204; 406) further comprises another power supply contact (224) adapted to electrically couple to the power supply contact (218) of the first module (202; 404) to receive power therefrom.

6. The interlock system according, to any preceding claim, wherein the first power contact (114) is in electrical communication with a negative terminal (124) of the at least one cell (106).

7. The interlock system according to any preceding claim, wherein the second power contact (112) is in electrical communication with a positive terminal (126) of the at least one cell (106).

8. The interlock system according to any preceding claim, wherein the first module is a surgical assembly (2) having an end effector (14).

9. The interlock system according to claim 8, wherein the second module is a generator (28) attachable to the surgical assembly (2).

10. The interlock system according to claim 9, wherein the generator (28) is an ultrasonic generator adapted to supply mechanical vibration to the end effector (14).

11. The interlock system according to claim 9, wherein the generator (28) is an electrosurgical generator adapted to supply eleetr-osurgical energy to at least one electrode disposed on the end effector (14).

12. The interlock system according to claim 9, 10 or 11, wherein the surgical assembly (2) is a disposable instrument,

13. The interlock system according to any preceding claim, wherein the battery assembly further comprises a PNP transistor (304) having base (316), emitter (320), and collector (318) terminals, wherein the base terminal (316) is operatively coupled to the sense contact (310) of the battery assembly, the emitter terminal (320) is operatively coupled to a positive terminal (322) of the at least one cell (302), and the collector terminal (318) is operatively coupled to the second power contact (306) of the battery assembly.

14. The interlock system according to any preceding claim, further comprising a variable-impedance device electrically coupled between the second power contact (112, 306) and the at least one cell (106, 302) to provide an impedance therebetween, wherein the variable-impedance device is configured to set the impedance to a high impedance state in response to the standby signal.

15. The interlock system according to claim 14, wherein the variable-impedance device is one of a contactor, a relay, an insulated gate bipolar transistor, and a silicon-controlled rectifier.
